Europäisches Patentamt

European Patent Office

Office européen des brevets

(11) Veröffentlichungsnummer: **0 137 936**
**A1**

## (12) EUROPÄISCHE PATENTANMELDUNG

(21) Anmeldenummer: **84109199.4**

(22) Anmeldetag: **02.08.84**

(51) Int. Cl.⁴: **A 61 N 1/32**

(30) Priorität: **03.10.83 DE 3335849**

(43) Veröffentlichungstag der Anmeldung: **24.04.85**
**Patentblatt 85/17**

(84) Benannte Vertragsstaaten: **AT CH DE FR GB LI NL**

(71) Anmelder: **Siemens Aktiengesellschaft, Berlin und München Wittelsbacherplatz 2, D-8000 München 2 (DE)**

(72) Erfinder: **Welgert, Kurt, Grillparzerstrasse 26, D-8510 Fürth (DE)**

(54) Interferenzstromtherapiegerät.

(57) Ein solches elektromedizinisches Gerät dient zur Erzeugung niederfrequenter Interferenzströme aus wenigstens zwei mittelfrequenten Strömen geringer Frequenzabweichung oder periodischer Phasenverschiebung. Zur Reduzierung des Aufwandes für die Erzeugung und Einstellung zahlreicher unterschiedlicher Formen von Wechsel- und Interferenzströmen ist jedem Patientenstromkreis (A, B) ein Umsetzer (31, 32) mit einem digitalen Stromformspeicher (311, 321) und einem nachgeschalteten Digitalanalogwandler (312, 322) zugeordnet. In jedem Stromformspeicher sind die Amplitudenwerte an äquidistanten Punkten der gewünschten Stromform digital abgespeichert und werden durch eine Steuereinrichtung (2) durch Ausgabe der zugeordneten Adressen periodisch ausgelesen. Die Frequenz des Wechselstromes ist dabei durch die Folgefrequenz der den Speicher abfragenden Adressen bestimmt. Die Adressen für den Stromformspeicher mindestens eines weiteren Patientenstromkreises werden mit Hilfe eines Adreßumsetzers (22) von den für den ersten Patientenstromkreis bestimmten Adressen abgeleitet, auf die hierzu Änderungswerte aufaddiert werden, die in einem Hilfsspeicher (221) abgelegt sind.

Siemens Aktiengesellschaft          Unser Zeichen
Berlin und München                  VPA 83 P 3339 E

— 1 —

## Interferenzstromtherapiegerät

Die Erfindung betrifft ein Interferenzstromtherapiegerät gemäß Oberbegriff von Anspruch 1.

Ein solches Gerät arbeitet mit mehreren an sich reizunwirksamen mittelfrequenten Strömen zwischen 1000 und 300.000 Hertz. Ein therapeutischer Effekt im Gewebe eines Patienten entsteht bei der Überlagerung solcher Ströme, wenn diese Ströme aufgrund einer geringen aber konstanten Frequenzdifferenz oder einer entsprechenden Phasenmodulation bei gleicher Frequenz einen Interferenzstrom erzeugen.

Bei einem aus der US-PS 40 71 033 bekannten Interferenzstromtherapiegerät der eingangs genannten Art besteht der Umsetzer jedes Kanals aus einem Amplitudenmodulator, mit dem die Amplitude der von einem Hauptoszillator gelieferten Rechteckimpulse dem gewünschten Verlauf entsprechend (rechteckförmig, sinusförmig etc.) verändert wird. Hierzu sind zusätzlich entsprechende Spannungsgeneratoren erforderlich, die eine derartige Modulationsspannung liefern.

Der Erfindung liegt die Aufgabe zugrunde, ein Interferenzstromtherapiegerät dieser Art zu vereinfachen, insbesondere wenn das Gerät in einfacher Weise auf sehr verschiedene Interferenzstromformen und -frequenzen einstellbar sein soll.

Ba 1 Sur / 24.07.1983

Bei der in Anspruch 1 gekennzeichneten Lösung dieser Aufgabe sind alle möglichen, sich periodisch wiederholenden Amplitudenwerte des Wechselstromes eines Kanals in einem adressierbaren, digitalen Stromformspeicher abgelegt. Die Frequenz des Wechselstromes und damit auch die Interferenzfrequenz zwischen den Wechselströmen mehrerer Kanäle hängt dabei vom Auslesetakt ab; die Form der Wechselströme sowie die Form der Schwebung ist durch die Adreßfolge bestimmt. Die Erfindung kommt daher ohne Modulator und insbesondere ohne zusätzlichen Generator für die verschiedenen Stromformen aus.

Zum Auslesen des Stromformspeichers jedes Kanals enthält die Steuereinrichtung vorzugsweise einen adressierbaren Adreßspeicher, in dem je Kanal mehrere auswählbare Adreßfolgen für unterschiedliche Formen und Frequenzen des Wechselstromes gespeichert sind.

Alternativ hierzu kann aber auch mit einem Adreßspeicher mit fest programmierter Adreßfolge gearbeitet werden. Eine Variation der Form des Wechselstromes jedes Kanals läßt sich dann dadurch erreichen, daß die aufeinanderfolgenden Amplitudenwerte aller möglichen Stromformen in einem Vorratsspeicher abgelegt und die Amplitudenwerte einer ausgewählten Stromform jeweils vor Beginn einer Behandlung in den Stromformspeicher jedes einzelnen Kanals übertragen werden.

Im Rahmen dieser Ausführungsform der Erfindung kann dann der Adreßspeicher als einfacher digitaler Zähler ausgebildet sein. Eine besonders einfache Ausführungsform der Erfindung erhält man in diesem Fall dadurch, daß die Adressen für einen zweiten Kanal von denen eines ersten Kanals durch numerische Veränderung gewonnen werden, wobei die Änderungswerte jeweils in einem Hilfsspeicher abgelegt sind und in einem vom Signalgenerator bestimmten Takt
ausgelesen werden.

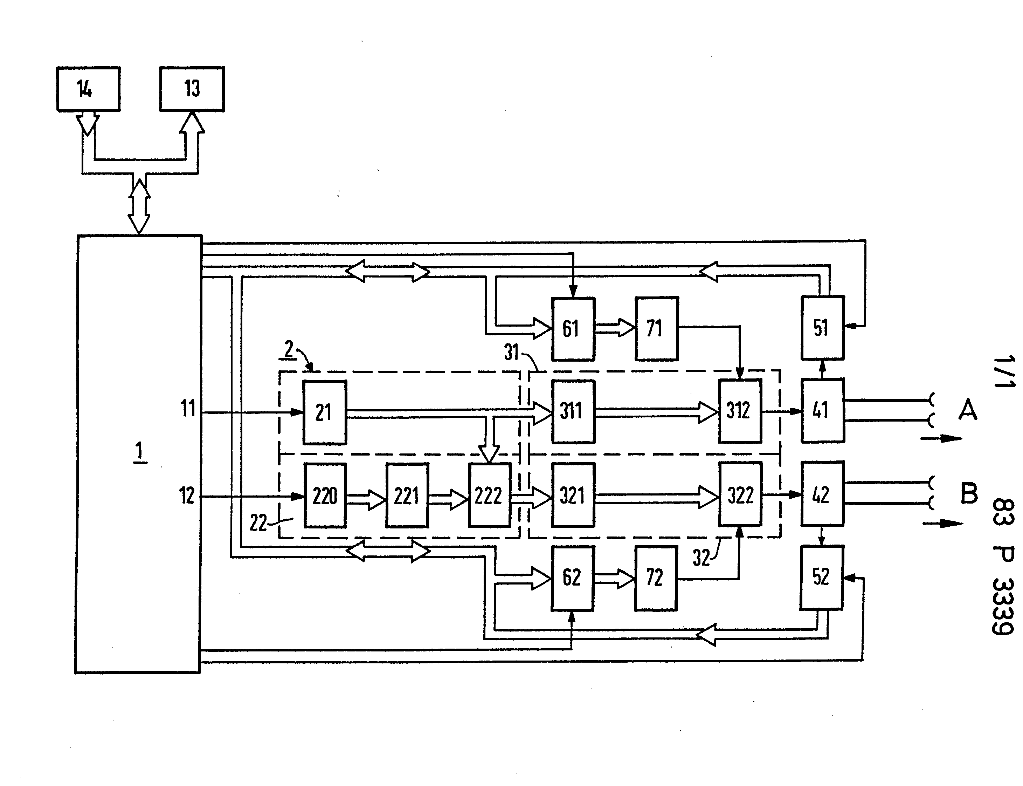

Ein Ausführungsbeispiel dieser Art wird anhand der Figur
näher erläutert, wobei alle digitalen Bausteine für 8 bit
breite Worte ausgelegt sind:

Jedem Patientenstromkreis A, B ist ein Kanal mit einem
Umsetzer 31, 32 und nachgeschaltetem Verstärker 41, 42
zugeordnet. Jeder Umsetzer besteht aus einem Stromformspeicher 311, 321 in Form eines ROM, dem ein Digitalanalogwandler 312 bzw. 322 nachgeschaltet ist.

Die Stromformspeicher 311, 312 der Umsetzer 31, 32 werden
von einer Steuereinrichtung 2 angesteuert, die an Impulsausgänge 11, 12 eines Mikroprozessors 1 angeschlossen
ist: Der Mikroprozessor liefert an diesen Ausgängen zwei
Taktimpulsfolgen, die von einem gemeinsamen Hauptoszillator, dem Taktgeber des Mikroprozessors abgeleitet sind.

In der Steuereinrichtung 2 ist ein Hauptzähler 21 vorgesehen, der durch die Taktimpulse am Impulsausgang 11 weitergeschaltet wird und dessen Ausgang einerseits mit dem
Adreßeingang des Stromformspeichers 311 des ersten Kanals
und andererseits mit dem einen Eingang eines binären
Addiergliedes 222 eines Adreßumsetzers 22 innerhalb der
Steuereinrichtung 2 verbunden ist. Die Ausgangsleitungen
des Addiergliedes 222 sind wiederum mit dem Adreßeingang
des Stromformspeichers 321 des Umsetzers 32 des zweiten
Kanals verbunden.

Der zweiten Eingang des Addiergliedes 222 liegt am Ausgang eines Hilfsspeichers 221 in Form eines ROM, dessen
Adressiereingang wiederum an einen Hilfszähler 220 angeschlossen ist, der durch die Taktimpulse des Impulsausganges 12 fortgeschaltet wird.

In den fortlaufend nummerierten Speicherplätzen der als ROM ausgebildeten Stromformspeicher 311, 321 sind die Amplitudenwerte an äquidistanten Punkten einer Periode des Wechselstromes gespeichert. Beim Durchschalten des Hauptzählers 21 im Takt der Taktimpulse am Impulsausgang 11 werden daher die Speicherplätze des Stromformspeichers 311 der Reihe nach abgefragt und die Amplitudenwerte an den nachgeschalteten Digitalanalogwandler 312 ausgegeben, an dessen Ausgang nach einem Durchlauf des Hauptzählers 21 eine Periode eines Wechselstromes mit der in 311 gespeicherten Form anliegt. Der Hauptzähler 21 wird nach einer Abfrage sämtlicher Adressen des Stromformspeichers durch den Mikroprozessor 1 wieder auf die erste Adresse zurückgestellt, so daß der Inhalt des Stromformspeichers 311 zyklisch ausgelesen wird und am Ausgang des Wandlers 312 der Wechselstrom der gewünschten Form anliegt. Seine Frequenz ist dabei durch die Folgefrequenz der Taktimpulse am Impulsausgang 11 des Mikroprozessors 1 bestimmt.

Prinzipiell gleichartig wird der Wechselstrom im Patientenstromkreis B erzeugt, wobei die Form dieses Wechselstromes durch die in dem Stromformspeicher 321 abgelegten Amplitudenwerte bestimmt und seine Frequenz genauso groß ist wie die des Wechselstromes im Patientenstromkreis A. Zwischen den beiden Wechselströmen besteht jedoch eine Phasenverschiebung, die sich daraus ergibt, daß aus den beiden Stromformspeichers 311, 321 gleichzeitig unterschiedliche Adressen ausgelesen werden: Die Adresse des Stromformspeichers 321 ergibt sich nämlich als Summe der Adresse von 311 und einem aus dem Hilfsspeicher 221 ausgelesenen Änderungswert.

Bei konstantem Änderungswert ergäbe sich auch eine konstanze Phasendifferenz zwischen den beiden Wechselströmen und somit keine Schwebung. Um die therapeutisch wichtige Schwebung mit der gewünschten Schwebungsfrequenz und einer bestimmten Hüllkurve zu erreichen, wird der dem Addierglied 222 zugeführte Änderungswert periodisch verändert, was eine entsprechende Phasenmodulation des Wechselstroms im zweiten Kanal und damit eine Schwebung zwischen den Wechselströmen der beiden Kanälen zur Folge hat. Die Form dieser Schwebung ist dabei durch die in dem Hilfsspeicher 221 unter aufeinanderfolgenden Adressen abgelegten Änderungswerte bestimmt. Die Schwebungsfrequenz resultiert aus der Folgefrequenz der am Impulsausgang 12 gelieferten Taktimpulse, die den Hilfszähler 220 weiterschalten und damit den Hilfsspeicher 221 periodisch abfragen.

Jedem Kanal ist noch ein Regelkreis zugeordnet, der dafür sorgt, daß der über einen Analogdigitalwandler 51, 52 gewonnene, digitalisierte Istwert des Patientenstromes stets mit dem in einem RAM-Speicher 61, 62 abgespeicherten momentanen Sollwert übereinstimmt. Eine eventuelle Abweichung wird nach Umwandlung in einen Analogwert mit Hilfe eines Digitalanalogwandlers 71, 72 dem Digitalanalogwandler 312, 322 im Umsetzer 31, 32 des zugehörigen Kanals zugeführt, der hierzu als multiplizierender Wandler ausgebildet ist.

Dem Mikroprozessor 1 ist noch ein Anzeigefeld 13 zur Überwachung und Anzeige sowie ein Bedienfeld 14 zur Einstellung der Parameter der Wechselströme und der Schwebung zugeordnet. Sofern die Stromformspeicher 311, 321 und der Hilfsspeicher 221 als RAM-Speicher ausgeführt sind, kann das Bedienfeld 14 einen Vorratsspeicher für alle auswählbaren Formen der Wechselströme und der Interferenzströme sowie einen Programmspeicher zur Übertragung der jeweils ausgewählten Form in die erwähnten Speicher mit Hilfe des Mikroprozessors enthalten.

## Begriffsliste

|           |                             |
|-----------|-----------------------------|
|           | Interferenzstromtherapiegerät |
|           | Kanal                       |
|           | Wechselstrom                |
| A, B      | Patientenstromkreis         |
| 1         | Signalgenerator             |
| 11, 12    | Impulsausgang               |
|           | Taktimpulsfolge             |
|           | Taktimpulse                 |
|           | Hauptoszillator             |
| 13        | Anzeigefeld                 |
| 14        | Bedienfeld                  |
|           | Vorratsspeicher             |
|           | Programmspeicher            |
| 2         | Steuereinrichtung           |
| 21        | Hauptzähler                 |
|           | Adreßspeicher               |
|           | Adreßfolgen                 |
| 22        | Adreßumsetzer               |
| 220       | Hilfszähler                 |
| 221       | Hilfsspeicher               |
| 222       | Addierglied                 |
|           | Änderungswert .             |
| 31, 32    | Umsetzer                    |
| 311, 321  | Stromformspeicher           |
| 312, 322  | Digitalanalogwandler        |
| 41, 42    | Verstärker                  |
| 51, 52    | Analogdigitalwandler        |
| 61, 62    | RAM-Speicher                |
| 71, 72    | Digitalanalogwandler        |

0137936

Patentansprüche

1. Interferenzstromtherapiegerät

- mit mindestens zwei Kanälen zur Erzeugung von Wechselströmen unterschiedlicher Frequenz für daran anschließbare Patientenstromkreise (A, B),

- mit einem Signalgenerator (1), der je Kanal einen Impulsausgang (11, 12) hat und daran eine Taktimpulsfolge
liefert, wobei die Taktimpulsfolgen der Kanäle unterschiedliche Folgefrequenzen haben und untereinander
synchronisiert sind,

- und mit einem Umsetzer (31, 32) je Kanal, der ausgangsseitig den Wechselstrom liefert und eingangsseitig mit
einem der Impulsausgänge (11, 12) des Signalgenerators
(1) verbunden ist,

d a d u r c h   g e k e n n z e i c h n e t ,

- daß jeder Umsetzer (31, 32) einen digitalen, adressierbaren Stromformspeicher (311, 321) und einen diesem
nachgeschalteten Digitalanalogwandler (312, 322) hat,

- daß in dem Stromformspeicher (311, 321) die aufeinanderfolgenden, periodisch wiederkehrenden Amplitudenwerte
des Wechselstromes in digitaler Form gespeichert sind,

- und daß zwischen dem Signalgenerator (1) und dem Umsetzer (31, 32) jedes Kanals eine Steuereinrichtung (2)
angeordnet ist, die jeden Stromformspeicher durch Ausgabe von Adressen periodisch abfragt.

2. Interferenzstromtherapiegerät nach Anspruch 1,
d a d u r c h  g e k e n n z e i c h n e t , daß die
Steuereinrichtung einen programmierbaren Adreßspeicher
hat, in dem je Kanal mehrere auswählbare Adreßfolgen für
unterschiedliche Formen und Frequenzen des Wechselstromes
gespeichert sind.

3. Interferenzstromtherapiegerät nach Anspruch 1,
d a d u r c h  g e k e n n z e i c h n e t , daß die
Steuereinrichtung einen Adreßspeicher für mehrere auswählbare Adreßfolgen für unterschiedliche Formen und Frequenzen des Wechselstromes eines ersten Kanals enthält,
und daß jedem weiteren Kanal ein Adreßumsetzer zugeordnet
ist, der die an den ersten Kanal ausgegebenen Adressen in
einstellbarer Weise periodisch verändert.

4. Interferenzstromtherapiegerät nach Anspruch 3,
d a d u r c h  g e k e n n z e i c h n e t ,

daß die aufeinanderfolgenden, periodisch wiederkehrenden Amplitudenwerte des Wechselstromes jedes Kanals in
dem zugeordneten Stromformspeicher unter fortlaufend
nummerierten Adressen gespeichert sind,

daß als Adreßspeicher ein digitaler Hauptzähler (31)
dient, dem eingangsseitig Taktimpulse eines Hauptoszillators im Signalgeber (1) zugeführt werden,

daß der Adreßumsetzer (22) ein Addierglied (222) enthält, das auf die von dem Hauptzähler (21) gelieferten
Adressen für den ersten Kanal Änderungswerte addiert,
die in einem Hilfsspeicher (221) abgelegt sind,

und daß der Hilfsspeicher (221) durch einen Hilfszähler
(220) gesteuert wird, der seinerseits durch vom Hauptoszillator abgeleitete Taktimpulse weitergeschaltet
wird.


5.    Interferenzstromtherapiegerät nach einem der Ansprüche 1 bis 4, d a d u r c h   g e k e n n z e i c h -
n e t , daß der Signalgenerator mit Hauptoszillator Teil
eines Mikroprozessors (1) ist.


6.    Interferenzstromtherapiegerät nach einem der Ansprüche 1 bis 5, d a d u r c h   g e k e n n z e i c h -
n e t ,  daß jedem Kanal ein Regelkreis zugeordnet ist, in
dem der digitalisierte Istwert des Wechselstromes im Patientenstromkreis mit einem digitalen Sollwert verglichen
und ein davon abhängiger, analoger Differenzwert gebildet
wird,

und daß der Digitalanalogwandler (312, 322) im Umsetzter
(31, 32) jedes Kanals als multiplizierender Wandler ausgebildet ist, dem als Multiplikator der analoge Differenzwert zugeführt wird.


7.    Interferenzstromtherapiegerät nach einem der Ansprüche 1 bis 3, d a d u r c h   g e k e n n z e i c h n e t ,
daß ein digitaler Vorratsspeicher vorgesehen ist, in dem
die auswählbaren Stromformen gespeichert sind, und daß
ein Programmspeicher vorgesehen ist, in dem die zur Übertragung einer ausgewählten Stromform von dem Vorratsspeicher in die Stromformspeicher oder zugeordneten Adreßspeicher erforderlichen Schritte abgespeichert sind.

0137936

## EINSCHLÄGIGE DOKUMENTE

EP 84109199.4

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE - A1 - 3 049 530 (DEUTSCHE NE-MECTRON) <br><br> * Seite 1, Anspruch 1; Seite 2, Ansprüche 2-6; Fig. 1,2 * <br><br> -- | 1-5,7 | A 61 N 1/32 |
| A | DE - A1 - 2 937 984 (ROBERT BOSCH) <br><br> * Seite 1, Anspruch 1; Seite 2, Anspruch 3; Fig. 1,3 * <br><br> -- | 1-4 | |
| A | DE - A1 - 2 712 101 (SARABER) <br><br> * Seite 1, Anspruch 1; Seite 15, Absatz 2 - Seite 16, Absatz 1; Fig. 4 * <br><br> -- | 1 | |
| A | DE - B2 - 2 632 700 (DÖLKER) <br><br> * Spalte 1, Ansprüche 1-3; Spalte 3, Zeile 30 - Spalte 4, Zeile 18 * <br><br> ---- | 1,6 | RECHERCHIERTE SACHGEBIETE (Int. Cl.4) <br><br> A 61 N |

Der vorliegende Recherchenbericht wurde für alle Patentansprüche erstellt.

| Recherchenort | Abschlußdatum der Recherche | Prüfer |
|---|---|---|
| WIEN | 21-12-1984 | NEGWER |